# EUROPEAN PATENT SPECIFICATION

(11) **EP 4 360 759 B1**
(45) Date of publication and mention of the grant of the patent: **30.07.2025**
(21) Application number: 23168003.4
(22) Date of filing: 14.04.2023
(51) Int. Cl.: B01L 3/02, B01L 3/00, G01N 1/30, G01N 15/14, G01N 1/31, G01N 15/00, G01N 15/10

(54) **BIOLOGICAL PARTICLE ENRICHMENT APPARATUS AND PICO-DROPLET GENERATOR THEREOF**
VORRICHTUNG ZUR ANREICHERUNG BIOLOGISCHER PARTIKEL UND PICOTRÖPFCHENGENERATOR DAFÜR
APPAREIL D'ENRICHISSEMENT DE PARTICULES BIOLOGIQUES ET GÉNÉRATEUR DE PICOGOUTTELETTES ASSOCIÉ

(30) Priority: 27.10.2022 US 202263419834 P
(43) Date of publication of application: 01.05.2024
(73) Proprietor: Cytoaurora Biotechnologies, Inc., Zhubei City, Hsinchu County 30261 (TW)
(72) Inventor: HUANG, Chung-Er, 30261 Zhubei City, Hsinchu County (TW); CHEN, Sheng-Wen, 30261 Zhubei City, Hsinchu County (TW); HO, Hsin-Cheng, 30261 Zhubei City, Hsinchu County (TW); YE, Guang-Ci, 30261 Zhubei City, Hsinchu County (TW)
(74) Representative: Viering, Jentschura & Partner mbB Patent- und Rechtsanwälte

(56) References cited:
- EP-A1- 2 546 656
- WO-A1-2016/128480
- US-A- 5 819 948

## Description

### FIELD OF THE DISCLOSURE

The present disclosure relates to a biological particle apparatus, and more particularly to a biological particle enrichment apparatus and a pico-droplet generator thereof.

### BACKGROUND OF THE DISCLOSURE

EP 2 546 656 A1 discloses a discharge device (1) includes a discharge head (10) that discharges a liquid material (50) from a nozzle opening (11) connected to the cavity (14) by varying the internal pressure of a cavity (14) using an actuator (6). The discharge head (10) includes a monitoring portion (12) provided between the cavity (14) and the nozzle opening (11) and the discharge device (1) further includes a detection apparatus (7) that detects the number and/or form of the particle-like bodies (51) included in the liquid material (50) in the monitoring portion (12) of the discharge head (10) and a control unit (74) that drives the actuator (6) according to the detection result (7a) of the detection unit (7) to change the state of the particle-like bodies (51) included in the liquid material (50) of the monitoring portion (12).

An enrichment process is the first step in conventional research for biological particles, but the conventional enrichment process still requires a significant amount of manpower and has a problem that biological particles can be adhered to a pipe wall that is used in the process. Accordingly, how apparatuses or devices can be used to quickly and accurately implement the enrichment process has been one of the important areas of research and development in the relevant field.

### SUMMARY OF THE DISCLOSURE

In response to the above-referenced technical inadequacies, the present disclosure provides a biological particle enrichment apparatus and a pico-droplet generator thereof to effectively improve on the issues associated with conventional enrichment processes. According to the invention, a pico-droplet generator according to claim 1 and a biological particle enrichment apparatus according to claim 2 are provided. Some examples of said apparatus are defined in claims 3 to 15.

In order to solve the above-mentioned problems, one of the technical aspects adopted by the present disclosure is to provide a biological particle enrichment apparatus, which includes a pico-droplet generator and a biochip corresponding in position to the pico-droplet generator. The pico-droplet generator is configured to output a pico-droplet from a liquid specimen. The pico-droplet generator includes a container, a hollow needle, a first piezoelectric member, and a second piezoelectric member. The container is configured to receive the liquid specimen having a plurality of biological particles, and the container has a bottom side and a surrounding lateral side that is connected to the bottom side. The hollow needle includes a connection end and a free end that is opposite to the connection end. The connection end of the hollow needle is connected to the bottom side of the container so as to establish a fluid communication between the hollow needle and the container. Moreover, an inner diameter of the container is within a range from 5 times to 30 times of an inner diameter of the hollow needle. The first piezoelectric member has a ring-shaped arrangement and is disposed on the surrounding lateral side of the container. The first piezoelectric member is configured to enable the biological particles in the container to be moved along a direction away from the surrounding lateral side by vibrating the container. The second piezoelectric member is disposed on an outer surface of the hollow needle. The second piezoelectric member is configured to squeeze the hollow needle, so that the liquid specimen flows outwardly and passes through the free end to form the pico-droplet. The biological particles include at least one target biological particle, and the pico-droplet having the at least one target biological particle is defined as a target pico-droplet. The biochip is configured to carry the target pico-droplet and to capture the at least one target biological particle in the target pico-droplet.

In order to solve the above-mentioned problems, another one of the technical aspects adopted by the present disclosure is to provide a pico-droplet generator of a biological particle enrichment apparatus. The pico-droplet generator includes a container, a hollow needle, a first piezoelectric member, and a second piezoelectric member. The container is configured to receive a liquid specimen having a plurality of biological particles. The container has a bottom side and a surrounding lateral side that is connected to the bottom side. The hollow needle includes a connection end and a free end that is opposite to the connection end. The connection end of the hollow needle is connected to the bottom side of the container so as to establish a fluid communication between the hollow needle and the container. Moreover, an inner diameter of the container is within a range from 5 times to 30 times of an inner diameter of the hollow needle. The first piezoelectric member has a ring-shaped arrangement and is disposed on the surrounding lateral side of the container. The first piezoelectric member is configured to enable the biological particles in the container to be moved along a direction away from the surrounding lateral side by vibrating the container. The second piezoelectric member is disposed on an outer surface of the hollow needle. The second piezoelectric member is configured to squeeze the hollow needle, so that the liquid specimen flows outwardly and passes through the free end to form a pico-droplet.

Therefore, any one of the biological particle enrichment apparatus and the pico-droplet generator thereof in the present disclosure can be provided to enable the biological particles to be moved toward a center of the container through a vibration of the first piezoelectric member, thereby preventing the biological particles from being adhered to inner walls of the container and completing the enrichment process of the target biological particle.

These and other aspects of the present disclosure will become apparent from the following description of the embodiment taken in conjunction with the following drawings and their captions, although variations and modifications therein may be affected without departing from the scope of the novel concepts of the disclosure.

### BRIEF DESCRIPTION OF THE DRAWINGS

The described embodiments may be better understood by reference to the following description and the accompanying drawings, in which:
FIG. 1 is a flowchart of a biological particle analysis method according to an embodiment of the present disclosure;
FIG. 2 is a schematic perspective view of an identifying system according to the embodiment of the present disclosure;
FIG. 3 is a schematic side view showing the identifying system operated to implement a staining step;
FIG. 4 is a schematic view showing a fluorescence staining process of FIG. 3;
FIG. 5 is a schematic side view showing the identifying system operated to implement an analyzing step;
FIG. 6 is a schematic cross-sectional view showing a part of FIG. 5;
FIG. 7 is a schematic view showing a camera device of the identifying system operated to take a real-time image of a pico-droplet generator;
FIG. 8 is a schematic side view showing the identifying system operated to implement a capturing step;
FIG. 9 is a schematic cross-sectional view showing a part of FIG. 8;
FIG. 10 is a schematic view showing a target biological particle captured by a biochip of the identifying system;
FIG. 11 is a schematic view showing the target biological particle captured by the biochip of the identifying system with another configuration;
FIG. 12 is a schematic view showing the pico-droplet generator of the identifying system outputting an abandoned pico-droplet;
FIG. 13 is a schematic side view showing the identifying system operated to implement a washing step;
FIG. 14 is a schematic side view showing the identifying system operated to implement a characterization expressing step; and
FIG. 15 is a partial schematic cross-sectional view of the camera device and the pico-droplet generator of the identifying system.

### DETAILED DESCRIPTION OF THE EXEMPLARY EMBODIMENTS

The present disclosure is more particularly described in the following examples that are intended as illustrative only since numerous modifications and variations therein will be apparent to those skilled in the art. Like numbers in the drawings indicate like components throughout the views. As used in the description herein and throughout the claims that follow, unless the context clearly dictates otherwise, the meaning of "a," "an" and "the" includes plural reference, and the meaning of "in" includes "in" and "on." Titles or subtitles can be used herein for the convenience of a reader, which shall have no influence on the scope of the present disclosure.

The terms used herein generally have their ordinary meanings in the art. In the case of conflict, the present document, including any definitions given herein, will prevail. The same thing can be expressed in more than one way. Alternative language and synonyms can be used for any term(s) discussed herein, and no special significance is to be placed upon whether a term is elaborated or discussed herein. A recital of one or more synonyms does not exclude the use of other synonyms. The use of examples anywhere in this specification including examples of any terms is illustrative only, and in no way limits the scope and meaning of the present disclosure or of any exemplified term. Likewise, the present disclosure is not limited to various embodiments given herein. Numbering terms such as "first," "second" or "third" can be used to describe various components, signals or the like, which are for distinguishing one component/signal from another one only, and are not intended to, nor should be construed to impose any substantive limitations on the components, signals or the like.

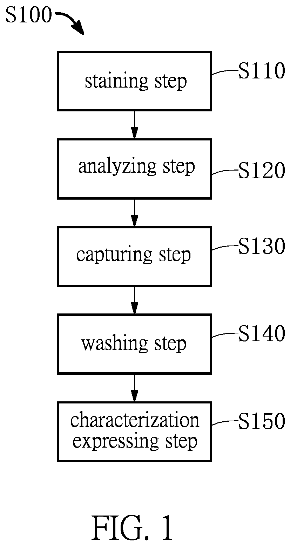

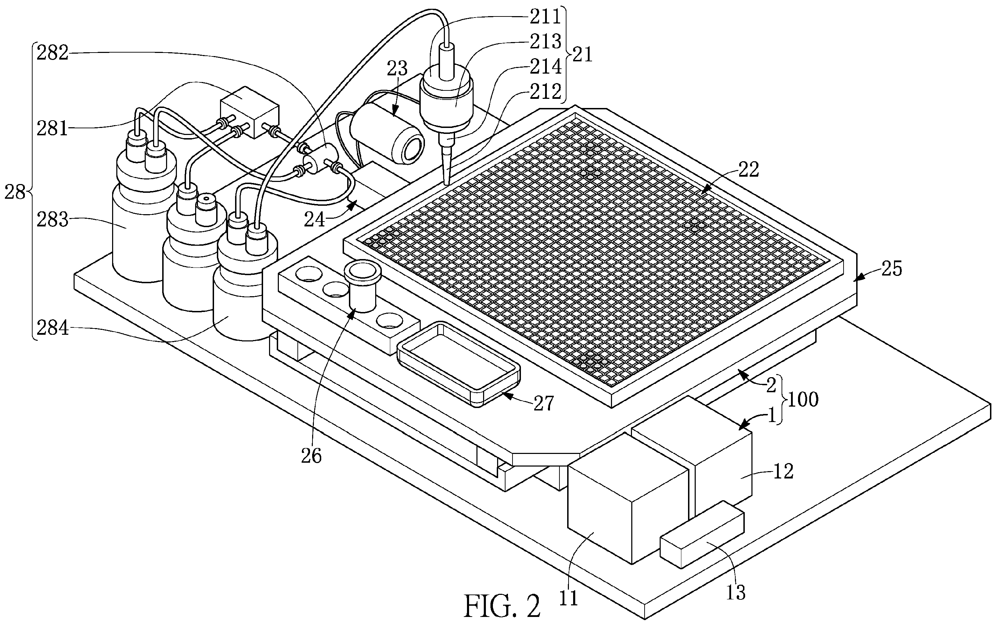

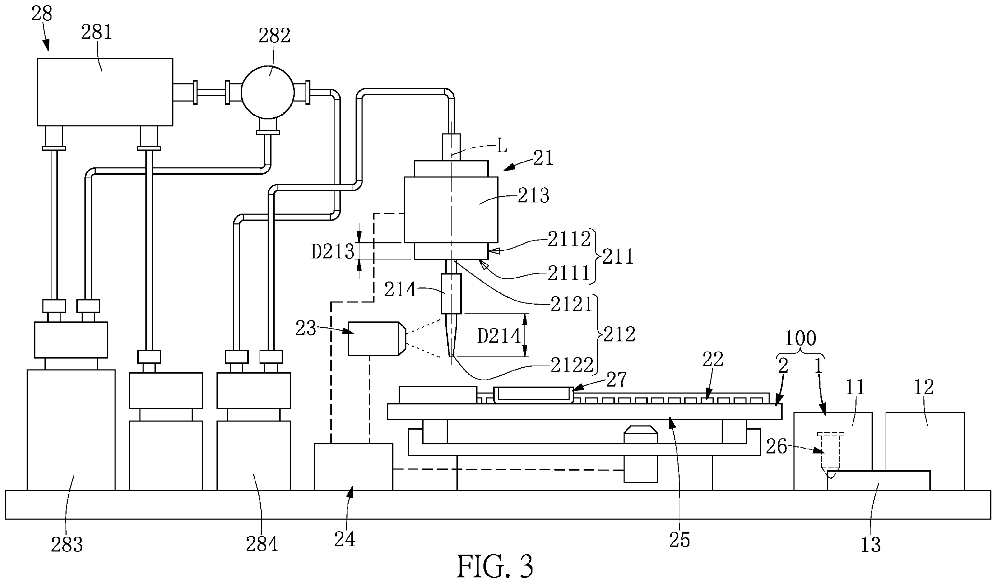

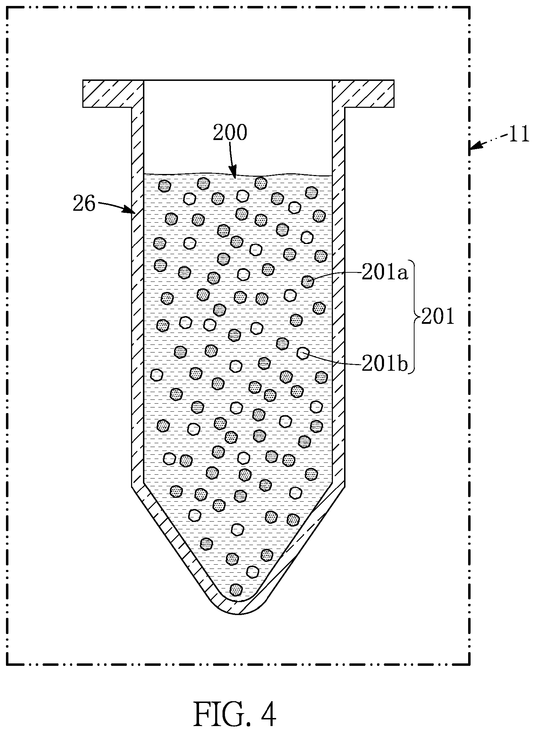

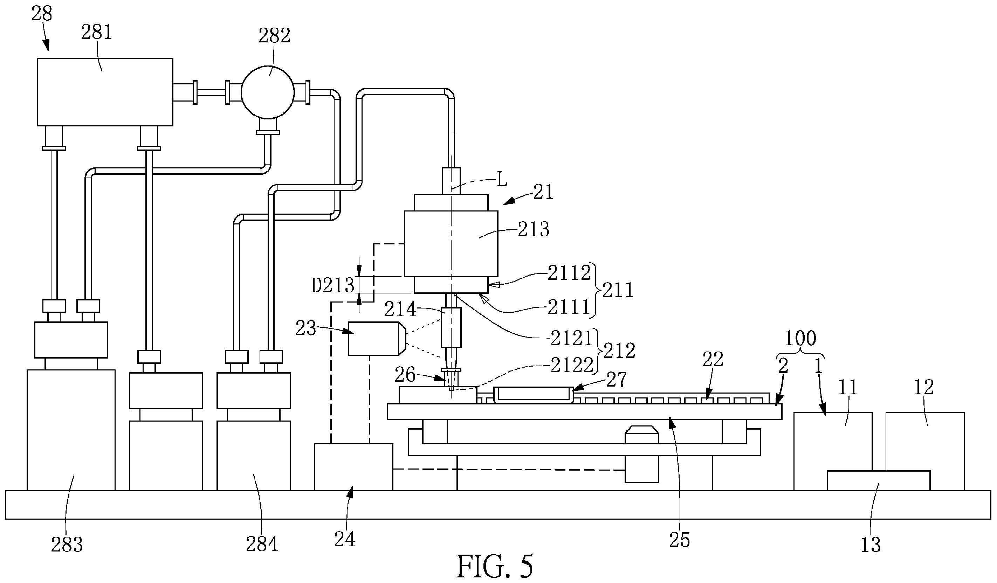

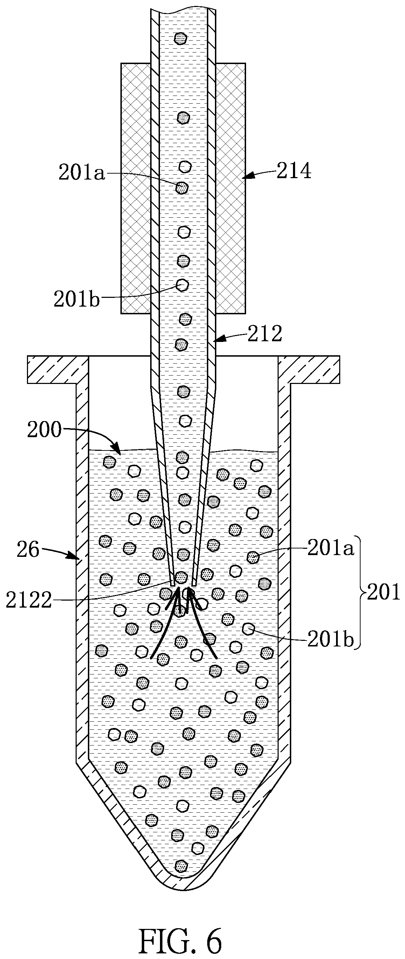

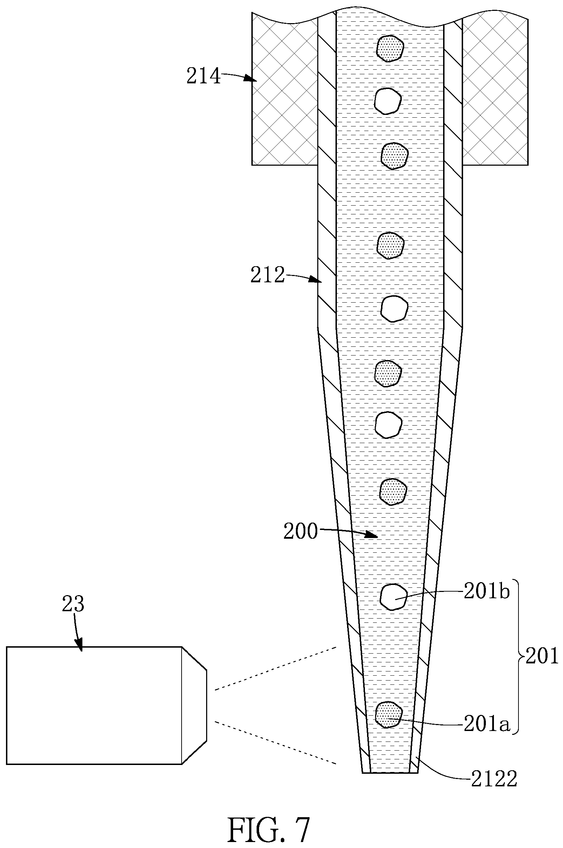

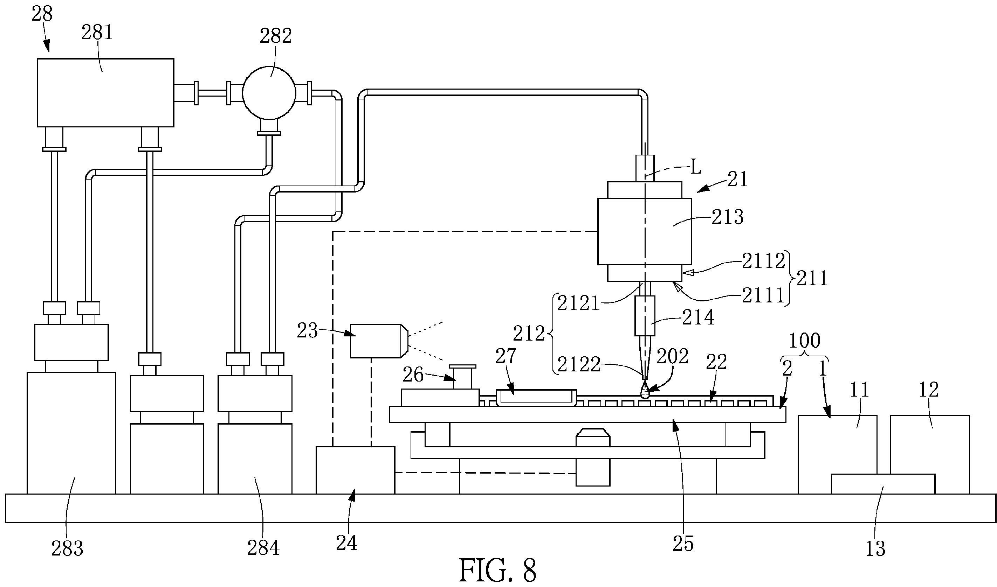

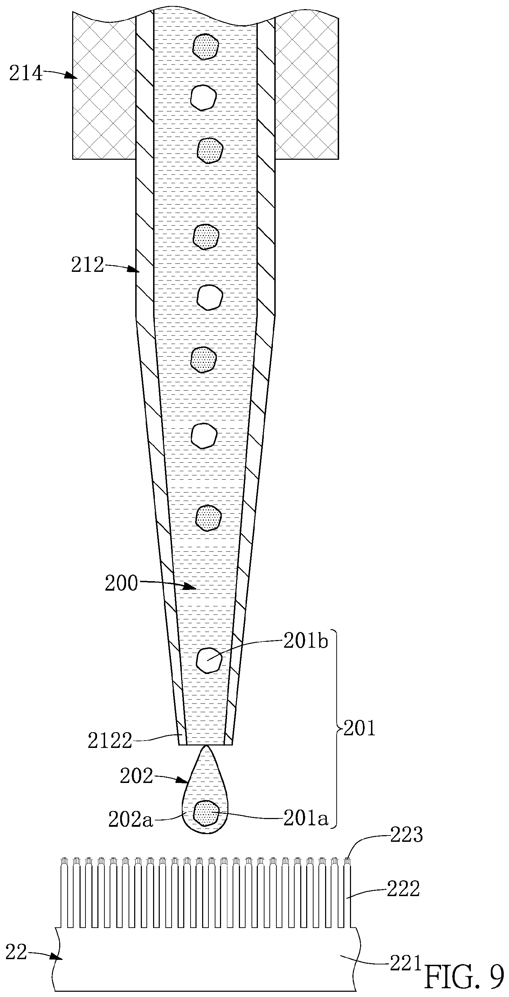

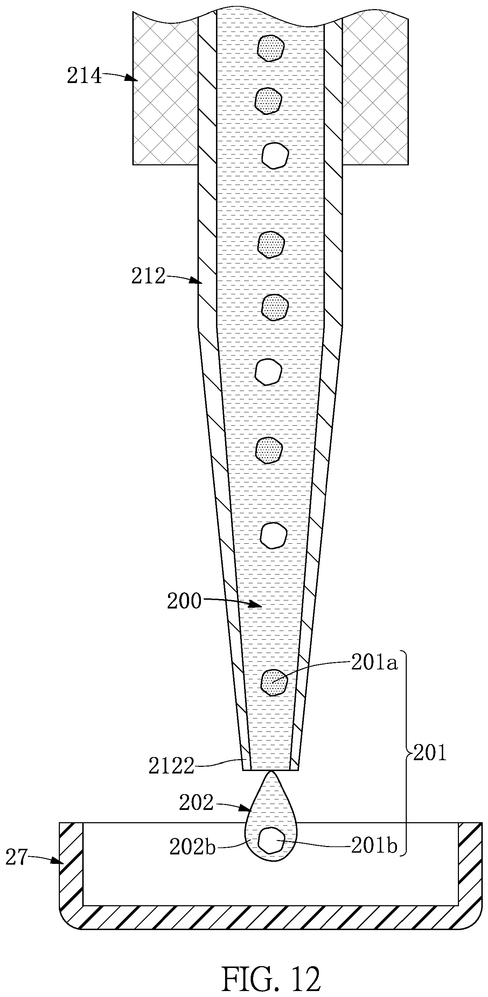

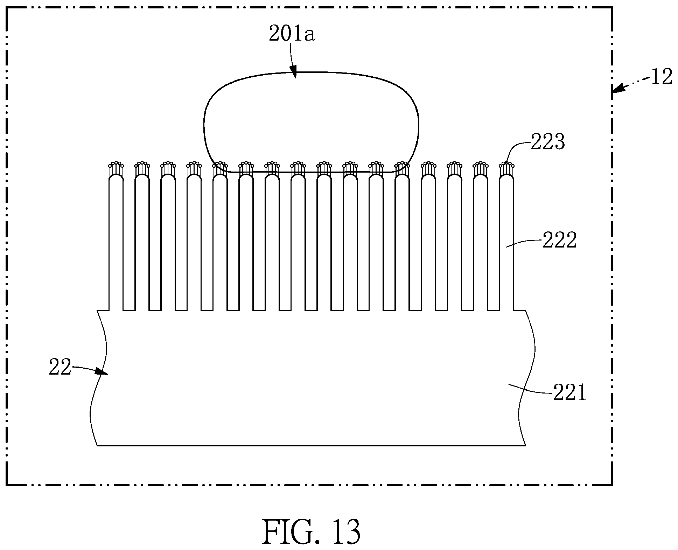

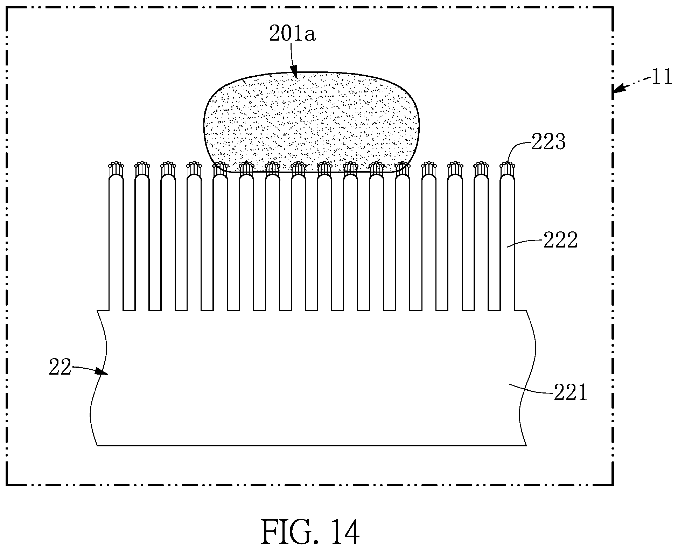

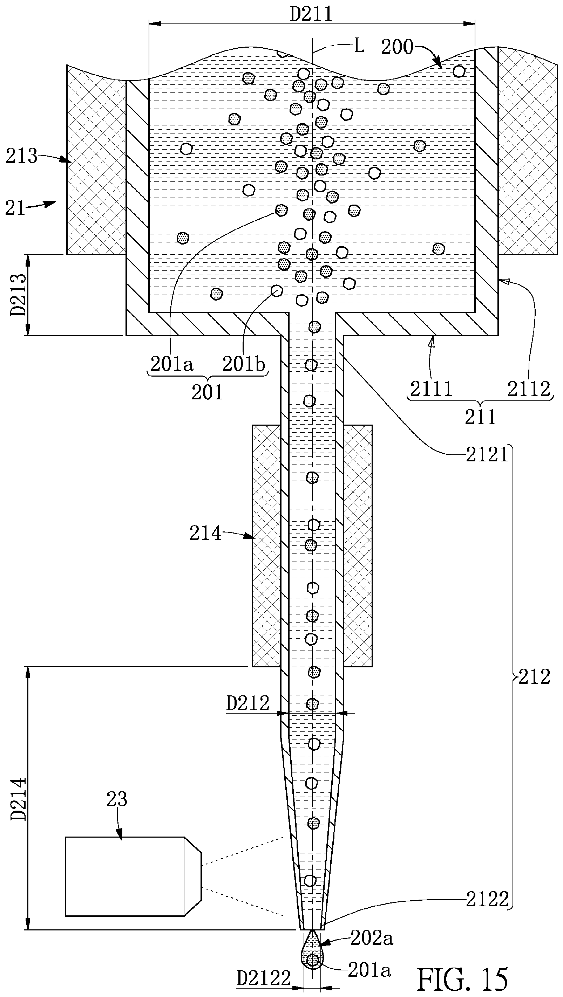

Referring to FIG. 1 to FIG. 15, a first embodiment of the present disclosure is provided. As shown in FIG. 1 and FIG. 2, the present embodiment provides a biological particle analysis method S100 and an identifying system 100. The identifying system 100 is applied to the biological particle analysis method S100, and the specific configuration of the identifying system 100 can be adjusted or changed according to design requirements, but the present disclosure is not limited thereto. For example, in other embodiments of the present disclosure not shown in the drawings, the biological particle analysis method S100 can be implemented through a structure other than the identifying system 100.

In the present embodiment, the identifying system 100 includes a multi-fluorescence staining apparatus 1, a pico-droplet generator 21, a camera device 23, and a biochip 22 that is provided to connect an operation of the multi-fluorescence staining apparatus 1 and an operation of the pico-droplet generator 21. The multi-fluorescence staining apparatus 1 includes a staining device 11, a washing device 12 arranged adjacent to the staining device 11, and a recording device 13 (e.g., a camera) that corresponds in position to the staining device 11, but the present disclosure is not limited thereto.

As shown in FIG. 1 and FIG. 3 to FIG. 14, the biological particle analysis method S100 in the present embodiment sequentially includes a staining step S110, an analyzing step S120, a capturing step S130, a washing step S140, and a characterization expressing step S150, thereby effectively completing an enrichment process and multiple biological characterization expressions for at least one target biological particle 201a that is selected from a liquid specimen 200 having a plurality of biological particles 201. In other words, any method not completely implementing the above steps in sequence is different from the biological particle analysis method S100 of the present embodiment.

As shown in FIG. 1, FIG. 3, and FIG. 4, the staining step S110 is implemented by fluorescence staining the liquid specimen 200 through a fluorescence staining process, so that at least one of the biological particles 201 becomes a fluorescence color and is defined as the at least one target biological particle 201a. In other words, the biological particles 201 are divided into the at least one target biological particle 201a and other non-target biological particles 201b.

In the present embodiment, the liquid specimen 200 is received in the specimen container 26, and the fluorescence staining process is implemented to the liquid specimen 200 in the specimen container 26 through the staining device 11 of the multi-fluorescence staining apparatus 1, but the present disclosure is not limited thereto. For example, in other embodiments of the present disclosure not shown in the drawings, the liquid specimen 200 in the specimen container 26 can be stained in other manners without using the staining device 11. Accordingly, the liquid specimen 200 being fluorescence stained is received in the specimen container 26.

Moreover, the liquid specimen 200 can be a body fluid from an animal (e.g., e.g., blood, lymph, saliva, ascites, or urine), and the at least one target biological particle 201a can be a specific type of cell, such as circulating tumor cells (CTCs), fetal nucleated red blood cells (FNRBCs), exosome, virus, or bacterium, but the present disclosure is not limited thereto. For example, in other embodiments of the present disclosure not shown in the drawings, the liquid specimen 200 can be chosen from plants.

As shown in FIG. 1 and FIG. 5 to FIG. 7, the analyzing step S120 is implemented by accommodating the liquid specimen 200 being fluorescence stained into the pico-droplet generator 21, and using the camera device 23 to take a real-time image of the liquid specimen 200 in the pico-droplet generator 21. In the present embodiment, the pico-droplet generator 21 can be used to receive the liquid specimen 200 therein from the specimen container 26, and an immediate position of the at least one target biological particle 201a having the fluorescence color can be obtained by using the camera device 23 to take the real-time image.

As shown in FIG. 1 and FIG. 8 to FIG. 12, the capturing step S130 is implemented by using the pico-droplet generator 21 to output a target pico-droplet 202a having the at least one target biological particle 201a onto the biochip 22 according to the real-time image. In other words, pico-droplets 202 generated from the liquid specimen 200 in the pico-droplet generator 21 in the present embodiment are divided into the target pico-droplet 202a and an abandoned pico-droplet 202b not having the target biological particle 201a. It should be noted that the pico-droplet 202, which has the non-target biological particle 201b but does not have the target biological particle 201a, is also defined as the abandoned pico-droplet 202b.

Specifically, whether the pico-droplet 202 outputted from the pico-droplet generator 21 has the at least one target biological particle 201a, which can be confirmed according to the real-time image, so that the pico-droplet 202 can be accurately placed onto the biochip 22 each time for enabling the at least one target biological particle 201a in the target pico-droplet 202a to be captured by the biochip 22, and the abandoned pico-droplet 202b is placed into an abandoned container 27, thereby effectively completing the enrichment process of the at least one target biological particle 201a.

It should be noted that the biochip 22 in the present embodiment is manufactured through a semi-conductor process, and the specific structure of the biochip 22 can be adjusted or changed according to design requirements. In order to clearly describe the biochip 22, the following description describes one kind of the biochip 22 having a better capturing effect, but the present disclosure is not limited thereto.

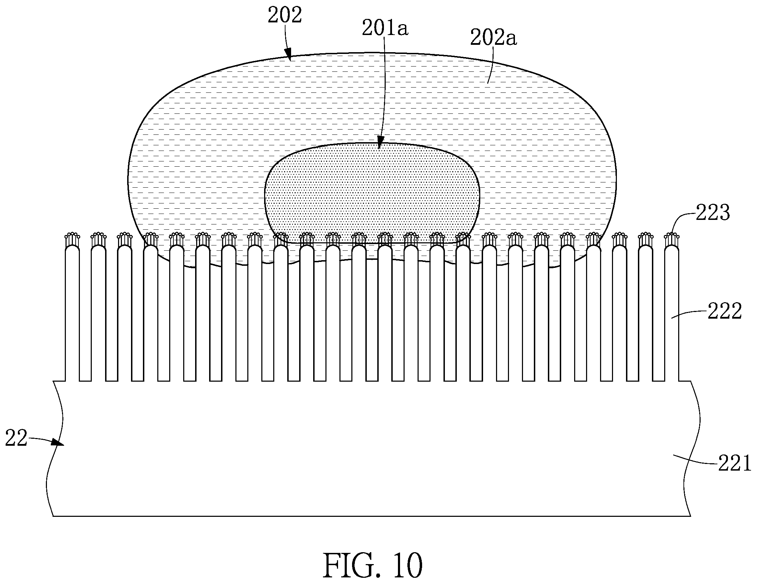

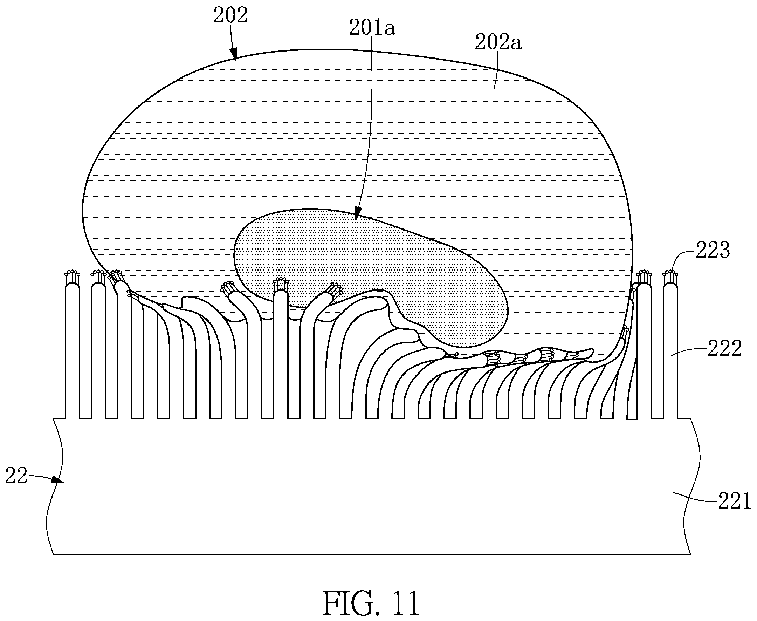

Specifically, as shown in FIG. 10 and FIG. 11, the biochip 22 is configured to carry the target pico-droplet 202a, and is capable of capturing the at least one target biological particle 201a in the target pico-droplet 202a. The biochip 22 includes a bottom layer 221, a plurality of capturing arms 222 connected to the bottom layer 221 and spaced apart from each other, and a surface modification layer 223 that is formed on ends of the capturing arms 222. In the capturing step S 130, the biochip 22 is configured to capture the at least one target biological particle 201a through the capturing arms 222 and the surface modification layer 223.

Specifically, the surface modification layer 223 is preferably an arginylglycylaspartic acid (RGD) peptide layer, thereby facilitating capturing of the at least one target biological particle 201a in a chemical bonding manner, but the present disclosure is not limited thereto. Moreover, as shown in FIG. 11, at least two of the capturing arms 222 of the biochip 22 are elastically swingable with respect to the bottom layer 221 and are capable of clamping (or pinching) the at least one target biological particle 201a, thereby facilitating capturing of the at least one target biological particle 201a in a physical manner.

As shown in FIG. 1, FIG. 2, and FIG. 13, the washing step S140 is implemented by removing the fluorescent color of the at least one target biological particle 201a captured by the biochip 22 through a washing process. In the present embodiment, the washing process is implemented to the at least one target biological particle 201a captured by the biochip 22 through the washing device 12 of the multi-fluorescence staining apparatus 1.

As shown in FIG. 1, FIG. 2, and FIG. 14, the characterization expressing step S150 is implemented by fluorescence staining the at least one target biological particle 201a captured by the biochip 22 for N number of times through the fluorescence staining process and the washing process, and using the recording device 13 to obtain a plurality of fluorescence images respectively corresponding to N kinds of biological characterization expressions. In other words, each fluorescence staining of the at least one target biological particle 201a is related to (or corresponds to) one of the kinds of the biological characterization expressions. In the present embodiment, N is a positive integer within a range from 2 to 50, and N is preferably within a range from 4 to 28, but the present disclosure is not limited thereto.

In addition, the biochip 22 can be moved among different devices (e.g., the staining device 11, the washing device 12, and the carrying platform 25) through manpower or automation (e.g., by a robotic arm), but the present disclosure is not limited thereto.

In summary, the capturing step S130 of the biological particle analysis method S100 in the present embodiment can be implemented to achieve the enrichment effect by being cooperated with the staining step S110 and the analyzing step S 120. Moreover, the biological particle analysis method S 100 in the present embodiment can be implemented to effectively connect the capturing step S 130, the washing step S 140, and the characterization expressing step S150 by using the biochip 22 to firmly capture the at least one target biological particle 201a. Accordingly, the fluorescence images respectively corresponding to the N kinds of biological characterization expressions can be obtained from the liquid specimen 200 through the biological particle analysis method S100, thereby facilitating any evaluation and determination of the at least one target biological particle 201a. In addition, the biological particle analysis method S100 can be implemented such that the fluorescence images are overlapped with each other, thereby obtaining a biological characterization of the at least one target biological particle 201a.

As shown in FIG. 1, FIG. 2, and FIG. 15, the above description describes the steps S110 - S150 of the biological particle analysis method S100 provided by the present embodiment, and in order to more clearly illustratee the present embodiment, a biological particle enrichment apparatus 2 that is cooperated with the multi-fluorescence staining apparatus 1 for jointly implementing the biological particle analysis method S 100 is further described, but the present disclosure is not limited thereto. For example, in other embodiments of the present disclosure not shown in the drawings, the biological particle enrichment apparatus 2 can be independently used (e.g., sold) or can be used in cooperation with other components.

In the present embodiment, the biological particle enrichment apparatus 2 includes the pico-droplet generator 21, the biochip 22 and the camera device 23 both corresponding in position to the pico-droplet generator 21, a controlling device 24 electrically coupled to the pico-droplet generator 21 and the camera device 23, a carrying platform 25 being capable of carrying the biochip 22 and corresponding in position to the pico-droplet generator 21, the specimen container 26 and the abandoned liquid container 27 both disposed on the carrying platform 25, and a pressure balance mechanism 28 that is connected to the pico-droplet generator 21, but the present disclosure is not limited thereto.

For example, in other embodiments of the present disclosure not shown in the drawings, types and a quantity of interior components of the biological particle enrichment apparatus 2 can be adjusted or changed according to design requirements, and the pico-droplet generator 21 can be independently used (e.g., sold) or can be used in cooperation with other components (e.g., the biochip 22).

The following description describes the structure and connection relationship of each component of the biological particle enrichment apparatus 2. In addition, part of the components (e.g., the biochip 22) of the biological particle enrichment apparatus 2 is described in the above description and will be omitted herein for the sake of brevity.

The pico-droplet generator 21 includes a container 211, a hollow needle 212 being in fluid communication with the container 211, a first piezoelectric member 213 disposed on the container 211, and a second piezoelectric member 214 that is disposed on the hollow needle 212. The container 211 is configured to receive the liquid specimen 200 and to transmit the liquid specimen 200 into the hollow needle 212, so that the hollow needle 212 can receive the liquid specimen 200 therein.

Specifically, the container 211 includes a bottom side 2111 and a surrounding lateral side 2112 that is connected to the bottom side 2111. The hollow needle 212 includes a connection end 2121 and a free end 2122 that is opposite to the connection end 2121. The connection end 2121 of the hollow needle 212 is (perpendicularly) connected to the bottom side 2111 of the container 211 so as to establish a fluid communication between the hollow needle 212 and the container 211. Moreover, an inner diameter D211 of the container 211 is within a range from 5 times to 30 times of an inner diameter D212 of the hollow needle 212. Specifically, the inner diameter D212 of the hollow needle 212 in the present embodiment that is measured is a portion other than the free end 2122 and is preferably within a range from 300 µm to 700 µm, and the free end 2122 of the hollow needle 212 in the present embodiment has an inner diameter D2122 being within a range from 30 µm to 150 µm, but the present disclosure is not limited thereto.

The first piezoelectric member 213 has a ring-shaped arrangement and is (annularly) disposed on the surrounding lateral side 2112 of the container 211, and the first piezoelectric member 213 in the present embodiment can be referred to as a top piezoelectric member 213. The first piezoelectric member 213 is configured to vibrate the container 211 for enabling the biological particles 201 in the container 211 to be moved along a direction away from the surrounding lateral side 2112. In other words, the first piezoelectric member 213 is configured to vibrate the container 211 for enabling the biological particles 201 in the container 211 to be arranged along a predetermined path.

Specifically, the predetermined path is preferably located along a central axis L of the hollow needle 212 (and a direction of gravity). Along a direction parallel to the central axis L, the first piezoelectric member 213 is spaced apart from the connection end 2121 by a distance D213 that is within a range from 0.2 cm to 2 cm. Moreover, the first piezoelectric member 213 is connected to and covers 20% to 85% of an area of the surrounding lateral side 2112 of the container 211. In addition, according to design requirements, the first piezoelectric member 213 can be a single one-piece structure having an annular shape or can be a structure having multiple components in an annular arrangement.

The second piezoelectric member 214 is disposed on an outer surface of the hollow needle 212, and the second piezoelectric member 214 in the present embodiment can be referred to as a bottom piezoelectric member 214. The second piezoelectric member 214 is configured to squeeze the hollow needle 212, so that the liquid specimen 200 flows outwardly and passes through the free end 2122 to form the pico-droplet 202. The pico-droplet 202 generated by the pico-droplet generator 21 can be further defined as a target pico-droplet 202a having the at least one target biological particle 201a or an abandoned pico-droplet 202b not having the target biological particle 201a.

Specifically, along a direction parallel to the central axis L, the second piezoelectric member 214 is spaced apart from the free end 2122 by a distance D214 that is within a range from 0.2 cm to 2 cm. The second piezoelectric member 214 is connected to and covers 20% to 85% of an area of the outer surface of the hollow needle 212. In addition, according to design requirements, the second piezoelectric member 214 can be a single one-piece structure having an annular shape or can be a structure having multiple components in an annular arrangement.

Furthermore, the pressure balance mechanism 28 is connected to the container 211, and the pressure balance mechanism 28 is configured to enable the liquid specimen 200 in the container 211 and the hollow needle 212 to be maintained at a predetermined pressure. The pressure balance mechanism 28 in the present embodiment is described as follows, but the present disclosure is not limited thereto. The pressure balance mechanism 28 includes an air pump 281, a switch 282 connected to the air pump 281, a pressure balance bottle 283 being in fluid communication with the air pump 281 and the switch 282, and a liquid injection bottle 284 that is in fluid communication with the switch 282 and the container 211.

The biochip 22, the specimen container 26, and the abandoned liquid container 27 are disposed on the carrying platform 25, and the carrying platform 25 and the pico-droplet generator 21 are relatively movable to each other (e.g., the carrying platform 25 can be assembled with a multi-axis movable mechanism). Accordingly, the pico-droplet generator 21 (in the analyzing step S120) is moveable relative to the carrying platform 25 and is capable of sucking the liquid specimen 200 from the specimen container 26 through the free end 2122 of the hollow needle 212 (e.g., the liquid specimen 200 is sucked into the hollow needle 212 and the container 211). Moreover, the pico-droplet generator 21 is moveable relative to the carrying platform 25 so as to output the target pico-droplet 202a onto the biochip 22 and output the abandoned pico-droplet 202b into the abandoned liquid container 27.

The camera device 23 corresponds in position to the hollow needle 212, and the camera device 23 (in the analyzing step S120) is configured to take a real-time image of the liquid specimen 200 in the free end 2122. Moreover, the controlling device 24 is electrically coupled to the second piezoelectric member 214 and the camera device 23. According to the real-time image, the controlling device 24 (in the capturing step S130) is configured to drive the second piezoelectric member 214 when the at least one target biological particle 201a is located in the free end 2112, so that the second piezoelectric member 214 is driven to squeeze the hollow needle 212 to enable the liquid specimen 200 to flow outwardly and pass through the free end 2122 of the hollow needle 212 to form the target pico-droplet 202a.

In summary, the biological particle enrichment apparatus 2 of the present embodiment can be provided to enable the biological particles 201 to be moved toward a center of the container 211 through a vibration of the first piezoelectric member 213 (i.e., the top piezoelectric member 213), thereby preventing the biological particles 201 from being adhered to inner walls of the container 211 and completing the enrichment process of the target biological particle 201a.

Specifically, the biological particle analysis method S100 or the biological particle enrichment apparatus 2 in the present embodiment is provided with the cooperation between the camera device 23 and the second piezoelectric member 214 (i.e., the bottom piezoelectric member 214), so that according to the real-time image of the liquid specimen 200 in the free end 2122, the second piezoelectric member 214 can be driven to form the target pico-droplet 202a when the at least one target biological particle 201a is located in the free end 2122, thereby effectively completing the enrichment process of the target biological particle 201a.

### [Beneficial Effects of the Embodiment]

In conclusion, the capturing step of the biological particle analysis method in the present disclosure can be implemented to achieve the enrichment effect by being cooperated with the staining step and the analyzing step. Moreover, the biological particle analysis method in the present disclosure can be implemented to effectively connect the capturing step, the washing step, and the characterization expressing step by using the biochip to capture the at least one target biological particle. Accordingly, the fluorescence images respectively corresponding to multiple kinds of biological characterization expressions can be obtained from the liquid specimen through the biological particle analysis method, thereby facilitating any evaluation and determination of the at least one target biological particle.

In addition, the biological particle enrichment apparatus of the present disclosure can be provided to enable the biological particles to be moved toward a center of the container through a vibration of the first piezoelectric member (i.e., the top piezoelectric member), thereby preventing the biological particles from being adhered to inner walls of the container and completing the enrichment process of the target biological particle.

Specifically, the biological particle analysis method or the biological particle enrichment apparatus in the present disclosure is provided with the cooperation between the camera device and the second piezoelectric member (i.e., the bottom piezoelectric member), so that according to the real-time image of the liquid specimen in the free end, the second piezoelectric member can be driven to form the target pico-droplet when the at least one target biological particle is located in the free end, thereby effectively completing the enrichment process of the target biological particle.

The foregoing description of the exemplary embodiments of the disclosure has been presented only for the purposes of illustration and description and is not intended to be exhaustive or to limit the disclosure to the precise forms disclosed. Many modifications and variations are possible in light of the above teaching.

The embodiments were chosen and described in order to explain the principles of the disclosure and their practical application so as to enable others skilled in the art to utilize the disclosure and various embodiments and with various modifications as are suited to the particular use contemplated. Alternative embodiments will become apparent to those skilled in the art to which the present disclosure pertains without departing from its scope.

## Claims

1. A pico-droplet generator of a biological particle enrichment apparatus, comprising:
a container (211) configured to receive a liquid specimen (200) having a plurality of biological particles (201), wherein the container (211) has a bottom side (2111) and a surrounding lateral side (2112) that is connected to the bottom side (2111);
a hollow needle (212) including a connection end (2121) and a free end (2122) that is opposite to the connection end (2121), wherein the connection end (2121) of the hollow needle (212) is connected to the bottom side (2111) of the container (211) so as to establish a fluid communication between the hollow needle (212) and the container (211), and wherein an inner diameter (D211) of the container (211) is within a range from 5 times to 30 times of an inner diameter (D212) of the hollow needle (212); and
a second piezoelectric member (214) disposed on an outer surface of the hollow needle (212), wherein the second piezoelectric member (214) is configured to squeeze the hollow needle (212), so that the liquid specimen (200) flows outwardly and passes through the free end (2122) to form a pico-droplet (202);
**characterized by** a first piezoelectric member (213) having a ring-shaped arrangement and disposed on the surrounding lateral side (2112) of the container (211), wherein the first piezoelectric member (213) is configured to enable the biological particles (201) in the container (211) to be moved along a direction away from the surrounding lateral side (2112) by vibrating the container (211).

2. A biological particle enrichment apparatus, **characterized by** comprising:
a pico-droplet generator (21) according to claim 1 configured to output a pico-droplet (202) from a liquid specimen (200),
a biochip (22) corresponding in position to the pico-droplet generator (21), wherein the biological particles (201) include at least one target biological particle (201a), and the pico-droplet (202) having the at least one target biological particle (201a) is defined as a target pico-droplet (202a), and wherein the biochip (22) is configured to carry the target pico-droplet (202a) and to capture the at least one target biological particle (201a) in the target pico-droplet (202a).

3. The biological particle enrichment apparatus according to claim 2, wherein the first piezoelectric member (213) is configured to enable the biological particles (201) in the container (211) to be arranged along a predetermined path by vibrating the container (211).

4. The biological particle enrichment apparatus according to claim 3, wherein the predetermined path is located along a central axis (L) of the hollow needle (212).

5. The biological particle enrichment apparatus according to claim 4, wherein along a direction parallel to the central axis (L), the first piezoelectric member (213) is spaced apart from the connection end (2121) by a distance (D213) that is within a range from 0.2 cm to 2 cm.

6. The biological particle enrichment apparatus according to claim 4, wherein along a direction parallel to the central axis (L), the second piezoelectric member (214) is spaced apart from the free end (2122) by a distance (D214) that is within a range from 0.2 cm to 2 cm.

7. The biological particle enrichment apparatus according to claim 2, wherein the first piezoelectric member (213) is connected to and covers 20% to 85% of an area of the surrounding lateral side (2112) of the container (211), and the second piezoelectric member (214) is connected to and covers 20% to 85% of an area of the outer surface of the hollow needle (212).

8. The biological particle enrichment apparatus according to claim 2, wherein the biochip (22) includes a bottom layer (221), a plurality of capturing arms (222) connected to the bottom layer (221) and spaced apart from each other, and a surface modification layer (223) that is formed on ends of the capturing arms (222), and wherein the biochip (22) is configured to capture the at least one target biological particle (201a) through the capturing arms (222) and the surface modification layer (223).

9. The biological particle enrichment apparatus according to claim 2, further comprising a camera device (23) corresponding in position to the hollow needle (212), wherein the camera device (23) is configured to take a real-time image of the liquid specimen (200) in the free end (2122).

10. The biological particle enrichment apparatus according to claim 9, further comprising a controlling device (24) electrically coupled to the second piezoelectric member (214) and the camera device (23), wherein according to the real-time image, the controlling device (24) is configured to drive the second piezoelectric member (214) when the at least one target biological particle (201a) is located in the free end (2122).

11. The biological particle enrichment apparatus according to claim 2, further comprising a carrying platform (25) corresponding in position to the pico-droplet generator (21), wherein the biochip (22) is disposed on the carrying platform (25), and the carrying platform (25) and the pico-droplet generator (21) are movable relative to each other.

12. The biological particle enrichment apparatus according to claim 11, wherein the pico-droplet (202) not having the target biological particles (201a) is defined as an abandoned pico-droplet (202b), wherein the biological particle enrichment apparatus (2) further includes an abandoned liquid container (27) disposed on the carrying platform (25), and the pico-droplet generator (21) is movable relative to the carrying platform so as to output the target pico-droplet (202a) onto the biochip (22) and output the abandoned pico-droplet (202b) into the abandoned liquid container (27).

13. The biological particle enrichment apparatus according to claim 11, further comprising a specimen container (26) disposed on the carrying platform (25) and configured to receive the liquid specimen (200), wherein the pico-droplet generator (21) is moveable relative to the carrying platform (25) and is capable of sucking the liquid specimen (200) from the specimen container (26) through the free end (2122).

14. The biological particle enrichment apparatus according to claim 2, further comprising a pressure balance mechanism (28) connected to the container (211), wherein the pressure balance mechanism (28) is configured to enable the liquid specimen (200) in the container (211) and the hollow needle (212) to be maintained at a predetermined pressure.

15. The biological particle enrichment apparatus according to claim 14, wherein the pressure balance mechanism (28) includes:
an air pump (281);
a switch (282) connected to the air pump (281);
a pressure balance bottle (283) being in fluid communication with the air pump (281) and the switch (282); and
a liquid injection bottle (284) being in fluid communication with the switch (282) and the container (211).

## Patentansprüche

1. Pikotropfengenerator einer Biologische-Partikel-Anreicherungsvorrichtung, aufweisend:
einen Behälter (211), der konfiguriert ist, um eine flüssige Probe (200) mit einer Mehrzahl biologischer Partikel (201) aufzunehmen, wobei der Behälter (211) eine Unterseite (2111) und eine umgebende seitliche Seite (2112) aufweist, die mit der Unterseite (2111) verbunden ist,
eine Hohlnadel (212), die ein Verbindungsende (2121) und ein freies Ende (2122) aufweist, das dem Verbindungsende (2121) gegenüberliegt, wobei das Verbindungsende (2121) der Hohlnadel (212) mit der Unterseite (2111) des Behälters (211) verbunden ist, um eine Fluidverbindung zwischen der Hohlnadel (212) und dem Behälter (211) herzustellen, und wobei ein Innendurchmesser (D211) des Behälters (211) in einem Bereich vom 5-fachen bis zum 30-fachen eines Innendurchmessers (D212) der Hohlnadel (212) liegt, und
ein zweites piezoelektrisches Element (214), das an einer Außenfläche der Hohlnadel (212) angeordnet ist, wobei das zweite piezoelektrische Element (214) konfiguriert ist, um die Hohlnadel (212) zusammenzudrücken, so dass die flüssige Probe (200) nach außen fließt und durch das freie Ende (2122) hindurchgeht, um einen Pikotropfen (202) zu bilden,
**gekennzeichnet durch** ein erstes piezoelektrisches Element (213), das eine ringförmige Anordnung hat und an der umgebenden seitlichen Seite (2112) des Behälters (211) angeordnet ist, wobei das erste piezoelektrische Element (213) konfiguriert ist, um zu ermöglichen, dass die biologischen Partikel (201) im Behälter (211) durch In-Schwingung-Versetzen des Behälters (211) entlang einer Richtung weg von der umgebenden seitlichen Seite (2112) bewegt werden.

2. Biologische-Partikel-Anreicherungsvorrichtung, **dadurch gekennzeichnet, dass** sie aufweist:
einen Pikotropfengenerator (21) gemäß Anspruch 1, der konfiguriert ist, um einen Pikotropfen (202) aus einer flüssigen Probe (200) auszugeben,
einen Biochip (22), der in seiner Position mit dem Pikotropfengenerator (21) korrespondiert, wobei die biologischen Partikel (201) mindestens ein biologisches Zielpartikel (201a) aufweisen, und der Pikotropfen (202) mit dem zumindest einen biologischen Zielpartikel (201a) als Ziel-Pikotropfen (202a) definiert ist, und wobei der Biochip (22) konfiguriert ist, um den Ziel-Pikotropfen (202a) zu tragen und das mindestens eine biologische Zielpartikel (201a) in dem Ziel-Pikotropfen (202a) einzufangen.

3. Biologische-Partikel-Anreicherungsvorrichtung gemäß Anspruch 2, wobei das erste piezoelektrische Element (213) konfiguriert ist, um zu ermöglichen, dass die biologischen Partikel (201) in dem Behälter (211) durch In-Schwingung-Versetzen des Behälters (211) entlang eines vorbestimmten Pfades angeordnet werden.

4. Biologische-Partikel-Anreicherungsvorrichtung gemäß Anspruch 3, wobei sich der vorbestimmte Pfad entlang einer Mittelachse (L) der Hohlnadel (212) befindet.

5. Biologische-Partikel-Anreicherungsvorrichtung gemäß Anspruch 4, wobei entlang einer Richtung parallel zur Mittelachse (L) das erste piezoelektrische Element (213) vom Verbindungsende (2121) um einen Abstand (D213) beabstandet ist, der in einem Bereich von 0,2 cm bis 2 cm liegt.

6. Biologische-Partikel-Anreicherungsvorrichtung gemäß Anspruch 4, wobei entlang einer Richtung parallel zur Mittelachse (L) das zweite piezoelektrische Element (214) vom freien Ende (2122) um einen Abstand (D214) beabstandet ist, der in einem Bereich von 0,2 cm bis 2 cm liegt.

7. Biologische-Partikel-Anreicherungsvorrichtung gemäß Anspruch 2, wobei das erste piezoelektrische Element (213) mit 20 % bis 85 % eines Bereichs der umgebenden seitlichen Seite (2112) des Behälters (211) verbunden ist und diese bedeckt und das zweite piezoelektrische Element (214) mit 20 % bis 85 % eines Bereichs der Außenfläche der Hohlnadel (212) verbunden ist und diese bedeckt.

8. Biologische-Partikel-Anreicherungsvorrichtung gemäß Anspruch 2, wobei der Biochip (22) eine Bodenschicht (221), eine Mehrzahl von Erfassungsarmen (222), die mit der Bodenschicht (221) verbunden und voneinander beabstandet sind, und eine Flächenmodifikationsschicht (223) aufweist, die an Enden der Erfassungsarme (222) ausgebildet ist, und wobei der Biochip (22) konfiguriert ist, um das zumindest eine biologische Zielpartikel (201a) durch die Erfassungsarme (222) und die Flächenmodifizierungsschicht (223) zu erfassen.

9. Biologische-Partikel-Anreicherungsvorrichtung gemäß Anspruch 2, die ferner eine Kameravorrichtung (23) aufweist, die in ihrer Position mit der Hohlnadel (212) korrespondiert, wobei die Kameravorrichtung (23) konfiguriert ist, um ein Echtzeitbild der flüssigen Probe (200) in dem freien Ende (2122) aufzunehmen.

10. Biologische-Partikel-Anreicherungsvorrichtung gemäß Anspruch 9, die ferner eine Steuervorrichtung (24) aufweist, die elektrisch mit dem zweiten piezoelektrischen Element (214) und der Kameraeinrichtung (23) gekoppelt ist, wobei die Steuervorrichtung (24) gemäß dem Echtzeitbild konfiguriert ist, um das zweite piezoelektrische Element (214) anzusteuern, wenn sich das zumindest eine biologische Zielpartikel (201a) in dem freien Ende (2122) befindet.

11. Biologische-Partikel-Anreicherungsvorrichtung gemäß Anspruch 2, die ferner eine Trageplattform (25) aufweist, die in ihrer Position mit dem Pikotropfengenerator (21) korrespondiert, wobei der Biochip (22) auf der Trageplattform (25) angeordnet ist und die Trageplattform (25) und der Pikotropfengenerator (21) relativ zueinander bewegbar sind.

12. Biologische-Partikel-Anreicherungsvorrichtung gemäß Anspruch 11, wobei der Pikotropfen (202), der nicht die biologischen Zielpartikel (201a) aufweist, als ein Abfall-Pikotropfen (202b) definiert ist, wobei die Biologische-Partikel-Anreicherungsvorrichtung (2) ferner einen Flüssigabfallbehälter (27) aufweist, der auf der Trägerplattform (25) angeordnet ist, und der Pikotropfengenerator (21) relativ zu der Trägerplattform bewegbar ist, um den Ziel-Pikotropfen (202a) auf den Biochip (22) auszugeben und den Abfall-Pikotropfen (202b) in den Flüssigabfallbehälter (27) auszugeben.

13. Biologische-Partikel-Anreicherungsvorrichtung gemäß Anspruch 11, die ferner einen Probenbehälter (26) aufweist, der auf der Trägerplattform (25) angeordnet ist und konfiguriert ist, um die flüssige Probe (200) aufzunehmen, wobei der Pikotropfengenerator (21) relativ zur Trägerplattform (25) bewegbar ist und in der Lage ist, die flüssige Probe (200) aus dem Probenbehälter (26) durch das freie Ende (2122) hindurch anzusaugen.

14. Biologische-Partikel-Anreicherungsvorrichtung gemäß Anspruch 2, die ferner einen Druckausgleichsmechanismus (28) aufweist, der mit dem Behälter (211) verbunden ist, wobei der Druckausgleichsmechanismus (28) konfiguriert ist, um zu ermöglichen, dass die flüssige Probe (200) in dem Behälter (211) und der Hohlnadel (212) auf einem vorbestimmten Druck gehalten wird.

15. Biologische-Partikel-Anreicherungsvorrichtung gemäß Anspruch 14, wobei der Druckausgleichsmechanismus (28) aufweist:
eine Luftpumpe (281),
einen mit der Luftpumpe (281) verbundenen Schalter (282),
eine Druckausgleichsflasche (283), die in Fluidverbindung mit der Luftpumpe (281) und dem Schalter (282) ist, und
eine Flüssigkeitsinjektionsflasche (284), die in Fluidverbindung mit dem Schalter (282) und dem Behälter (211) ist.

## Revendications

1. Générateur de pico-gouttelettes d'un appareil d'enrichissement en particules biologiques, comprenant :
un récipient (211) configuré pour recevoir un échantillon liquide (200) contenant une pluralité de particules biologiques (201), dans lequel le récipient (211) comporte un côté inférieur (2111) et un côté latéral environnant (2112) qui est relié au côté inférieur (2111) ;
une aiguille creuse (212) comprenant une extrémité de connexion (2121) et une extrémité libre (2122) opposée à l'extrémité de connexion (2121), dans lequel l'extrémité de connexion (2121) de l'aiguille creuse (212) est reliée au côté inférieur (2111) du récipient (211) de manière à établir une communication fluidique entre l'aiguille creuse (212) et le récipient (211), et dans lequel un diamètre intérieur (D211) du récipient (211) est compris dans une plage allant de 5 fois à 30 fois un diamètre intérieur (D212) de l'aiguille creuse (212) ; et
un deuxième élément piézoélectrique (214) disposé sur une surface extérieure de l'aiguille creuse (212), dans lequel le deuxième élément piézoélectrique (214) est configuré pour presser l'aiguille creuse (212), de sorte que l'échantillon liquide (200) s'écoule vers l'extérieur et passe à travers l'extrémité libre (2122) pour former une pico-gouttelette (202) ;
**caractérisé par** un premier élément piézoélectrique (213) ayant une configuration annulaire et disposé sur le côté latéral environnant (2112) du récipient (211), dans lequel le premier élément piézoélectrique (213) est configuré pour permettre aux particules biologiques (201) dans le récipient (211) à être déplacées dans une direction s'éloignant du côté latéral environnant (2112) en faisant vibrer le récipient (211).

2. Appareil d'enrichissement en particules biologiques, **caractérisé en ce qu'**il comprend :
un générateur de pico-gouttelettes (21) selon la revendication 1, configuré pour émettre une pico-gouttelette (202) à partir d'un échantillon liquide (200),
une biopuce (22) correspondant en position au générateur de pico-gouttelettes (21), dans lequel les particules biologiques (201) comprennent au moins une particule biologique cible (201a), et la pico-gouttelette (202) contenant ladite au moins une particule biologique cible (201a) est définie comme une pico-gouttelette cible (202a), et dans lequel la biopuce (22) est configurée pour transporter la pico-gouttelette cible (202a) et pour capturer ladite au moins une particule biologique cible (201a) dans la pico-gouttelette cible (202a).

3. Appareil d'enrichissement en particules biologiques selon la revendication 2, dans lequel le premier élément piézoélectrique (213) est configuré pour permettre aux particules biologiques (201) dans le récipient (211) d'être disposées le long d'un trajet prédéterminé en faisant vibrer le récipient (211).

4. Appareil d'enrichissement en particules biologiques selon la revendication 3, dans lequel le trajet prédéterminé est situé le long d'un axe central (L) de l'aiguille creuse (212).

5. Appareil d'enrichissement en particules biologiques selon la revendication 4, dans lequel, le long d'une direction parallèle à l'axe central (L), le premier élément piézoélectrique (213) est espacé de l'extrémité de connexion (2121) d'une distance (D213) comprise dans une plage de 0,2 cm à 2 cm.

6. Appareil d'enrichissement en particules biologiques selon la revendication 4, dans lequel, le long d'une direction parallèle à l'axe central (L), le deuxième élément piézoélectrique (214) est espacé de l'extrémité libre (2122) d'une distance (D214) comprise dans une plage de 0,2 cm à 2 cm.

7. Appareil d'enrichissement en particules biologiques selon la revendication 2, dans lequel le premier élément piézoélectrique (213) est relié à et recouvre 20 % à 85 % d'une région du côté latéral environnant (2112) du récipient (211), et le deuxième élément piézoélectrique (214) est relié à et recouvre 20 % à 85 % d'une région de la surface extérieure de l'aiguille creuse (212).

8. Appareil d'enrichissement en particules biologiques selon la revendication 2, dans lequel la biopuce (22) comprend une couche inférieure (221), une pluralité de bras de capture (222) reliés à la couche inférieure (221) et espacés les uns des autres, et une couche de modification de surface (223) qui est formée sur des extrémités des bras de capture (222), et dans lequel la biopuce (22) est configurée pour capturer ladite au moins une particule biologique cible (201a) à travers les bras de capture (222) et la couche de modification de surface (223).

9. Appareil d'enrichissement en particules biologiques selon la revendication 2, comprenant en outre un dispositif de caméra (23) correspondant en position à l'aiguille creuse (212), dans lequel le dispositif de caméra (23) est configuré pour prendre une image en temps réel de l'échantillon liquide (200) dans l'extrémité libre (2122).

10. Appareil d'enrichissement en particules biologiques selon la revendication 9, comprenant en outre un dispositif de commande (24) couplé électriquement au deuxième élément piézoélectrique (214) et au dispositif de caméra (23), dans lequel, en fonction de l'image en temps réel, le dispositif de commande (24) est configuré pour entraîner le deuxième élément piézoélectrique (214) lorsque ladite au moins une particule biologique cible (201a) est située dans l'extrémité libre (2122).

11. Appareil d'enrichissement en particules biologiques selon la revendication 2, comprenant en outre une plate-forme de transport (25) correspondant en position au générateur de pico-gouttelettes (21), dans lequel la biopuce (22) est disposée sur la plate-forme de transport (25), et la plate-forme de transport (25) et le générateur de pico-gouttelettes (21) sont mobiles l'un par rapport à l'autre.

12. Appareil d'enrichissement en particules biologiques selon la revendication 11, dans lequel la pico-gouttelette (202) ne contenant pas les particules biologiques cibles (201a) est définie comme une pico-gouttelette abandonnée (202b), dans lequel l'appareil d'enrichissement en particules biologiques (2) comprend en outre un récipient de liquide abandonné (27) disposé sur la plate-forme de transport (25), et le générateur de pico-gouttelettes (21) est mobile par rapport à la plate-forme de transport de manière à émettre la pico-gouttelette cible (202a) sur la biopuce (22) et à émettre la pico-gouttelette abandonnée (202b) dans le récipient de liquide abandonné (27).

13. Appareil d'enrichissement en particules biologiques selon la revendication 11, comprenant en outre un récipient d'échantillon (26) disposé sur la plate-forme de transport (25) et configuré pour recevoir l'échantillon liquide (200), dans lequel le générateur de pico-gouttelettes (21) est mobile par rapport à la plate-forme de transport (25) et est capable d'aspirer l'échantillon liquide (200) du récipient d'échantillon (26) à travers l'extrémité libre (2122).

14. Appareil d'enrichissement en particules biologiques selon la revendication 2, comprenant en outre un mécanisme d'équilibrage de pression (28) relié au récipient (211), dans lequel le mécanisme d'équilibrage de pression (28) est configuré pour permettre à l'échantillon liquide (200) dans le récipient (211) et l'aiguille creuse (212) d'être maintenus à une pression prédéterminée.

15. Appareil d'enrichissement en particules biologiques selon la revendication 14, dans lequel le mécanisme d'équilibrage de pression (28) comprend :
une pompe à air (281) ;
un commutateur (282) relié à la pompe à air (281) ;
une bouteille d'équilibrage de pression (283) en communication fluidique avec la pompe à air (281) et le commutateur (282) ; et
une bouteille d'injection de liquide (284) en communication fluidique avec le commutateur (282) et le récipient (211).
